# EUROPEAN PATENT APPLICATION

(11) **EP 4 299 112 A1**
(43) Date of publication of application: **03.01.2024**
(21) Application number: 22759978.4
(22) Date of filing: 17.02.2022
(51) Int. Cl.: A61N 2/00, A61N 2/02, A61B 5/00, A61B 5/055, A61B 5/28, A61B 5/0245

(54) **BRAIN STIMULATING DEVICE INCLUDING NAVIGATION DEVICE FOR GUIDING POSITION OF COIL AND METHOD THEREOF**

(30) Priority: 26.02.2021 KR 20210026670
(71) Applicant: Advanced Technology & Communications Co., Ltd., Incheon 21635 (KR)
(72) Inventor: LEE, Jong-Won, Gunpo-si, Gyeonggi-do 15845 (KR); NA, Gi-Yong, Seoul 06375 (KR)
(74) Representative: Ström & Gulliksson AB
(86) International application number: PCT/KR2022/002353
(87) International publication number: WO 2022/182060

(57) **Abstract**

The present invention relates to a navigation device for guiding a coil location, a brain stimulating device comprising same, and a bio-navigation robot system. The navigation device for guiding a coil location according to an example embodiment of the present disclosure may comprise: a 3D camera unit for capturing an image of a user to obtain a 3D camera image; and a location data calculating unit for mapping the camera image to a 3D medical image including brain region information or mapping the 3D medical image to the camera image to obtain a mapped image and calculating location data of a brain region to be stimulated from the mapped image.

## Description

### Technical Field

The present disclosure relates to a brain stimulating device including a navigation device for guiding a position of a coil and a method thereof, and relates to a navigation device for sensing, selecting, or guiding a position of a coil using artificial intelligence (AI).

### Background Art

A magnetic stimulation method such as Transcranial Magnetic Stimulation (TMS) is a method of non-invasive stimulation of nerve cells in a brain using magnetic energy, and can perform psychiatric treatments such as for depression or neurological treatments such as for dementia by activating nerve cells by allowing a powerful magnetic field to pass through the skull near the head.

Conventionally, medical personnel such as nurses had to directly position a coil to generate a magnetic field in the location of a brain to be magnetically stimulated, and in this case, it took a lot of time to accurately position the coil, which may reduce the efficiency of treatments.

Additionally, before positioning the coil in the brain to be magnetically stimulated, on medical images such as MRI images, medical personnel such as doctors had to mark landmarks such as ears and eyebrows, and a brain region to actually be stimulated, and had to manually position the coil for a head of an actual user, based on the marked image.

This required separate time before the medical personnel could perform the treatment, and the medical personnel needed to take additional time to find a corresponding position immediately before performing the treatment, which was doubly time-consuming, and accordingly, there was a problem in that treatment time may be reduced to decrease treatment efficiency.

Therefore, there is a need for a navigation device for guiding a position of a magnetic stimulating device by detecting in real time an actual location of a brain to be magnetically stimulated and a position of a head corresponding to the location of the brain.

(Patent Document 1) KR10-2020-0139536 A

### Summary of Invention

### Technical Problem

An aspect of the present disclosure is to solve the conventional problem described above, and is to provide a brain stimulating device including a navigation device for guiding a position of a coil, for mapping an actual user's head image with a medical image and automatically guide a location of the brain to be magnetically stimulated and a position of a head corresponding thereto, and a method thereof.

### Solution to Problem

According to an aspect of the present disclosure, a navigation device for guiding a position of a coil may include: a 3D camera unit for capturing an image of a user to obtain a 3D camera image; and a location data calculating unit for mapping the camera image to a 3D medical image including brain region information or mapping the 3D medical image to the camera image to obtain a mapped image, and calculating real-time 3D coordinates as location data of a brain region to be stimulated from the mapped image.

Meanwhile, according to another aspect of the present disclosure, a method of controlling a navigation device for guiding a position of a coil may include: obtaining a 3D camera image by capturing an image of a user by a 3D camera unit; obtaining a mapped image by mapping the camera image to a 3D medical image including brain region information or mapping the 3D medical image to the camera image; and calculating real-time 3D coordinates as location data of a brain region to be stimulated in the mapped image by the location data calculating unit.

According to another aspect of the present disclosure, A computer-readable recording medium for recording a program for executing the method of controlling a navigation device for guiding a position of a coil on a computer.

### Advantageous Effects of Invention

According to an aspect of the present disclosure, medical personnel do not need to manually detect a position of brain be magnetically stimulated and a head position corresponding thereto before performing magnetic stimulation one by one, and both the time and manpower spent on position calculation may be used for treatment, improving user convenience and increasing accuracy to improve treatment efficiency.

Furthermore, since a position can be automatically calculated, magnetic stimulation may be performed at home as well as at a hospital, thereby increasing user convenience.

### Brief Description of Drawings

FIG. 1 is a flowchart illustrating a method of controlling a navigation device according to an example embodiment of the present disclosure.
FIG. 2 is a schematic block diagram illustrating a navigation device according to an example embodiment of the present disclosure.
FIG. 3 is an overall block diagram illustrating a bio-navigation robot system according to an example embodiment of the present disclosure.
FIG. 4 is a flowchart illustrating an operation of a facial recognition module according to an example embodiment of the present disclosure.
FIG. 5 is a flowchart illustrating an operation of a medical image analysis module according to an example embodiment of the present disclosure.
FIG. 6 is a flowchart illustrating an operation of an image mapping module according to an example embodiment of the present disclosure.
FIG. 7 is a flowchart illustrating an operation of guiding a position of a robot arm with a navigation device according to an example embodiment of the present disclosure.
FIG. 8 is a flowchart illustrating an operation of measuring a motor threshold measured by an MT measurement module according to an example embodiment of the present disclosure.
FIG. 9 is an overall block diagram of a brain stimulating device according to another example embodiment of the present disclosure.
FIG. 10 is a flowchart illustrating an operation of a facial recognition module according to another example embodiment of the present disclosure.
FIG. 11 is a flowchart illustrating a position of a coil of a helmet-type magnetic stimulating unit by a navigation device according to another example embodiment of the present disclosure.
FIG. 12 a block diagram schematically illustrating a navigation device according to another example embodiment of the present disclosure.
FIG. 13 is a view schematically illustrating an arrangement of a coil disposed in a helmet-type magnetic stimulating unit according to another example embodiment of the present disclosure.
FIG. 14 is a view illustrating a structure of a coil in a helmet-type magnetic stimulating unit according to another example embodiment of the present disclosure.

### Best Mode for Invention

Hereinafter, embodiments of the present disclosure will be described with reference to specific example embodiments and the attached drawings. The embodiments of the present disclosure may, however, be exemplified in many different forms and should not be construed as being limited to the specific embodiments set forth herein. In the drawings, the shapes and dimensions of elements may be exaggerated for clarity, and the same reference numerals will be used throughout to designate the same or like elements.

Hereinafter, a person who receives magnetic stimulation or controls a magnetic stimulation system to receive or receive the magnetic stimulation is defined as a user. A dementia patient or a cognitive impairment patient is one of the users, and the user is not limited to the patient. Additionally, the user includes a user who receives magnetic stimulation and a user who uses a device to receive the magnetic stimulation, and hereinafter, the user mainly denotes a user who receives the magnetic stimulation.

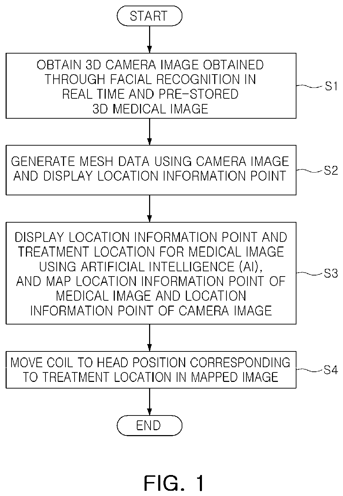

FIG. 1 is a flowchart schematically illustrating a control method in which a navigation device performs a position guide according to an example embodiment of the present disclosure.

As illustrated in FIG. 1, the navigation device may perform an operation of obtaining a 3D camera image obtained through facial recognition in real time and a pre-stored 3D medical image (S1), an operation of generating mesh data using the camera image and displaying a location information point (S2), an operation of displaying the location information point and a treatment location for the medical image using artificial intelligence (AI), and mapping the location information point of the medical image and the location information point of the camera image (S3), and an operation of moving a coil 160 to a head position corresponding to the treatment location in the mapped image (S4).

Specifically, in the operation of moving the coil 160 in S4, when location data for the treatment location is provided to the user, the coil 160 may be directly moved to the location data, the coil 160 may be automatically moved to the location data through a robot arm 161 connected to the coil 160, or the coil 160 disposed in the location data may be driven through a helmet-type magnetic stimulating unit 162.

Accordingly, a method of controlling a navigation device for guiding a position of the coil 160 according to an example embodiment of the present disclosure may include an operation of obtaining a 3D camera image by capturing an image of the user by a 3D camera unit 220, an operation of obtaining a mapped image by mapping the camera image to a 3D medical image including a brain region information by a location data calculating unit 231 or mapping the 3D medical image to the camera image, and an operation of calculating location data of a brain region to be stimulated in the mapped image by the location data calculating unit 231.

Specifically, the operation of obtaining a mapped image according to an example embodiment of the present disclosure may include an operation of detecting a location information point or a head that is a standard for the location data of the brain region to be stimulated using the camera image, an operation of detecting a mapping point or a head in a face region of the medical image, and the operation of mapping the camera image and the medical image by mapping the location information point and the mapping point or mapping the head of each image so as to obtain the location of the brain region to be stimulated and the head position corresponding to the brain region to be stimulated.

Image mapping may be performed in consideration of specific points and specific shapes by extracting location information points of a nose, a forehead and an earlobe, and shapes of external lines that form different heads for each user.

Furthermore, the operation of calculating location data of a brain region to be stimulated according to an example embodiment of the present disclosure may further include an operation of calibrating a preset zero point in the camera image or the medical image based on the mapped image, and generating real-time 3D coordinates in the mapped image based on the calibrated zero point, or an operation of detecting a motor cortex or the location of the brain region to be stimulated in the brain region of the medical image, and an operation of calculating real-time 3D coordinates for the position of the motor cortex or the brain region to be stimulated from the mapped image.

This is meant to calculate location data including coordinates of the brain region to be stimulated as well as location data of the motor cortex in order to calculate an optimal magnetic field strength for the user, and to secure information on a motor threshold (MT) by performing magnetic field stimulation.

Hereinafter, a navigation device performing the operations S1 to S3 will be described.

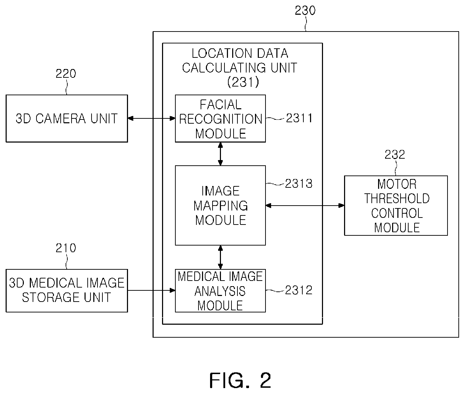

FIG. 2 is a schematic block diagram of a navigation device according to an example embodiment of the present disclosure, and the navigation device for performing the above-described control may include the following configuration.

According to an example embodiment of the present disclosure, the navigation device may include a 3D camera unit 220 for obtaining a 3D camera image by capturing an image of the user, and a location data calculating unit 231 for obtaining a mapped image by mapping the camera image to a 3D medical image including brain region information or mapping the 3D medical image to the camera image, and calculating location data of the brain region to be stimulated from the mapped image. The 3D medical image which is an image fixed to a 3D camera image of which the position varies according to the user's movement, may be mapped.

The 3D camera unit 220 may take an image of the user, and may capture a head including a brain region to be stimulated. The 3D camera unit 220 may capture an entire face including the head, or only a portion of an upper surface of the head corresponding to the brain region.

In this case, the user who takes an image of the head so that the head is well recognized may wear a hair contact band or a contact hat, and a separate recognition identification mark may be combined with the contact band or the contact hat so as to make it easier for the 3D camera unit 220 to recognize the contact band or the contact hat. The recognition identification mark may be made using a material or a receptor that can be easily recognized by the 3D camera unit 220, and the position of the head or the face may be accurately confirmed on the 3D camera image through the recognition identification mark.

Furthermore, as illustrated in FIG. 2, the navigation for guiding a position of the coil 160 according to an example embodiment of the present disclosure may include a 3D camera unit 220 for obtaining a 3D camera image by capturing a head in real time corresponding to the brain region to be stimulated by the user, a 3D medical image storage unit 210 for storing the 3D medical image of the user, a location data calculating unit 231 for receiving the camera image and the medical image, processing and mapping the images, and calculating location data of the brain region to be stimulated from the mapped image.

According to an example embodiment, the 3D medical image storage unit 210 may be omitted, and the user may provide a storage medium or recording medium in which the 3D medical image is stored, or receive a medical image from a separate server in which the 3D medical image is stored.

Specifically, the 3D camera unit 220 may be used to take an image of the user real time, preferably, the user's head, and in the chair 10, and may capture a current head of the user sitting on a chair 10 and a position change of the head according to a movement of the user. In this case, the entire head image including the face may be captured, and only a portion of the head having a brain region to be stimulated may be captured.

Furthermore, the 3D camera unit 220 is a camera capable of measuring depth, and may have n channels and may three-dimensionally capture the user's head through a plurality of n channels.

On the other hand, the 3D medical image storage unit 210 may store the 3D medical image of the user, and the 3D medical image refers to the International Standard Image (Digital Imaging and Communications in Medicine (DICOM)) of medical images, and may include, for example, an MRI image, a PET image, and a CT image.

The 3D medical image stored in the 3D medical image storage unit 210 according to an example embodiment of the present disclosure may be an image in which a location information point is already displayed, or a 3D medical image in which the location information point is not displayed, and the image may be input to the location data calculating unit 231 to automatically detect a location information point.

The location data calculating unit 231 may convert the input 3D medical image into mesh data and precisely divide the mesh data, and may detect a motor cortex or a brain region to be stimulated. The location information point may be automatically detected from the mesh data by obtaining the precisely divided mesh data.

The 3D medical image storage unit 210 may be included in the navigation device, or may exist as a separate server to deliver a 3D medical image to the navigation device.

Furthermore, as illustrated in FIG. 2, the location data calculating unit 231 according to an example embodiment of the present disclosure may include a facial recognition module 2311 for detecting a location information point or a head that is a standard of location data of a brain region to be stimulated using the camera image, a medical image analysis module 2312 for detecting a mapping point or the head in a face region of the medical image, and an image mapping module 2313 for mapping the location information point and the mapping point so as to obtain the location of the brain region to be stimulated and the position of the head corresponding to the brain region to be stimulated, or mapping the camera image and the medical image by mapping the head of each image, and obtaining the mapped image.

The location data calculating unit 231 may be included in a controller 230 of the navigation device as illustrated in FIG. 2, and may be included in a separate device.

Specifically, the facial recognition module 2311 may generate multiplex mesh data after image-processing a real-time camera image received from the 3D camera unit 220 or the 3D medical image stored in the 3D medical image storage unit 210.

A facial recognition algorithm may be applied to detect a characteristic portion of the face from the camera image received from the 3D camera unit 220, and by generating multiple mesh data according to the characteristic portion of the face, even if the user's face moves, the characteristic portion moves together, thus generating the multiple mesh data according to the user's movement.

Here, the characteristic portion of the face refers to a face protrusion or a face groove portion, and the face protrusion or the face groove portion may include a portion having a depth when capturing the user's face by a 3D camera according to a three-dimensional structure. In other words, the face protrusion or the face groove portion refers to a major configuration in which even if the user's face moves, when the position of the face protrusion or the face groove portion is determined, an overall face position of the user is determined together. For example, eyes, a nose, eyebrow bones, etc. may be a standard thereof.

Furthermore, the facial recognition module 2311 may detect the location information point based on the mesh data. Image analysis may be precisely performed by adjusting a mesh scale through the mesh data, and the location information point may be more easily detected based on the mesh data. In this case, the location information point is a preset reference point to easily detect the location of the brain region to be stimulated, and may refer to, for example, a head, a nose, a forehead, and an ear, and the location information point of a preset portion may include 3D coordinates of each portion.

Furthermore, the location information point may include a preset zero point, and the facial recognition module 2311 may detect the zero point in the mesh data.

The medical image analysis module 2312 illustrated in FIG. 2 may receive the 3D medical image stored from the 3D medical image storage unit 210 and analyze the medical image to detect mapping points of a head, a nose, a forehead, and ears.

Furthermore, the medical image analysis module 2312 may generate mesh data using the medical image, similarly to the facial recognition module 2311.

Accordingly, in the location data calculating unit 231, specifically, the facial recognition module 2311 and the medical image analysis module 2312 may generate the mesh data using the camera image in which the location information point is detected or the medical image in which the mapping point is detected, or generate the mesh data with the camera image or the medical image, and detect the location information point or the mapping point in the mesh data.

In other words, the accuracy and the precision of the location data may be increased by more precisely dividing the camera image and the medical image to calculate the location data.

The mesh data may be generated after displaying the location information point or the mapping point, or the location information point or the mapping point may be displayed after generating the mesh data, but the location information point or the mapping point may be displayed after generating the mesh data in order to increase the accuracy of the point.

Furthermore, the medical image analysis module 2312 may detect location data of the brain region to be stimulated in the 3D medical image and location data of the motor cortex.

The image mapping module 2312 illustrated in FIG. 2 may receive a camera image in which the location information point is displayed, from the facial recognition module 2311, and receive a medical image showing at least one of the location information point, the location data of the brain region to be stimulated, and the location data of the motor cortex, from the medical image analysis module 2312.

By mapping the location information point of the received camera image and the mapping point of the received medical image, the camera image and the medical image may be integrated into one image, and real-time 3D coordinates may be generated from the integrated image to obtain the location data of the brain region to be stimulated and the location data on the head corresponding to the brain region.

In other words, by mapping the medical image to the camera image based on the location information point, a zero coordinate and a 3D coordinate in which the brain region to be stimulated is disposed based on the zero coordinate may be generated based on the mapped image, and may be provided to a magnetic field generating device 100.

In this case, the zero point of each image may be twisted while matching the location information point and the mapping point, and the location data calculating unit 231 according to an example embodiment of the present disclosure may calibrate a preset zero point on the camera image or the medical image based on the mapped image, and may generate real-time 3D coordinates in the mapped image based on the calibrated zero point. When matching the mapping point to the location information point, the location data may be modified according to the mapped image by calibrating the zero point of the medical image to the zero point of the camera image.

Furthermore, the image mapping module 2312 may obtain the location data of the motor cortex and the location data on the head corresponding to the motor cortex and transmit the obtained data to a motor threshold control module 232, and the motor threshold control module 232 may measure the motor threshold (MT) through the received location data, or allow a motor threshold measurement module 1102 of the magnetic field generating device 100 to measure the motor threshold (MT) of the user by transmitting the location data of the motor cortex received to the magnetic field generating device 100.

That is, according to an example embodiment of the present disclosure, the image mapping module 2313 may include a medical image analysis module 2312 for detecting the motor cortex or the location of the brain region to be stimulated in the brain region of the medical image, and based on the mapped image, the 3D coordinates may be generated based on a zero coordinate, and the location of the 3D coordinates for the motor cortex or the location of the brain region to be stimulated by the magnetic field generating device may be calculated or provided in the mapped image.

Meanwhile, according to an example embodiment of the present disclosure, the location data calculating unit 231 may include an artificial intelligence (AI) model learned through artificial intelligence (AI) using data from a server.

Specifically, the location data calculating unit 231 may include a deep learning model learned through a deep learning algorithm using the data from the server, and the location data calculating unit 231 may match at least data among the location information points in the camera image varying depending on the user, the mapping point in the medical image varying depending on the user, and the matching point between the location information point and the mapping point, to user information, and store the matched data in the server, or may store a learning result of the location data calculating unit in the server. The camera image or the mapping point may be the mesh data.

The artificial intelligence model may learn the detected location information point or the detected mapping point stored in the server and may perform a location search for the location information point in the camera image and the mapping point in the medical image more accurately in short time, and may learn the matching point between the location information point and the mapping point, thus extracting the matching point more accurately and quickly at the time of integrating the camera image and the medical image.

Accordingly, according to an example embodiment of the present disclosure, at least one of the location information point in the camera image, the mapping point in the medical image, and the matching point between the location information point and the mapping point may be detected using the artificial intelligence model.

Because a size and a shape of the head or the brain vary depending on the user, the location of the location information point is changed all the times when generating the mesh data in the facial recognition module 2311, and the mapping point of the medical image in the medical image analysis module 2312 also is changed depending on the user.

Accordingly, before performing the magnetic field stimulation by the magnetic field generating device 100, a process of detecting the location information point or the mapping point in each image is necessarily performed, and accordingly, the time for performing stimulation may be reduced, which may result in poor stimulation efficiency, and inconvenience to the user.

Therefore, the user who has similar types of camera images or medical images by matching and storing the camera image and the location information point or by matching and storing the metical image and the mapping point may detect the location information point or the mapping point for each image more quickly and accurately.

For example, a deep learning model may be learned with MRI images showing brain treatment regions and location information points to be stimulated by doctors and other medical personnel, and internal weights may be modified according to the output, thus increasing the detection accuracy and speed of the location information point. This may make user-customized search possible, thereby maximizing magnetic stimulation efficiency.

As described above, existing data may be converted into database and stored in the server so that the deep learning model may use the existing accumulated data as learning data.

Furthermore, a cloud server for receiving and storing data stored in the server may be further provided.

When the user performs remote medical treatment or uses a navigation device for home use, the data may be stored in the cloud server so that the performed information may be checked and diagnosed by external medical personnel or external administrators.

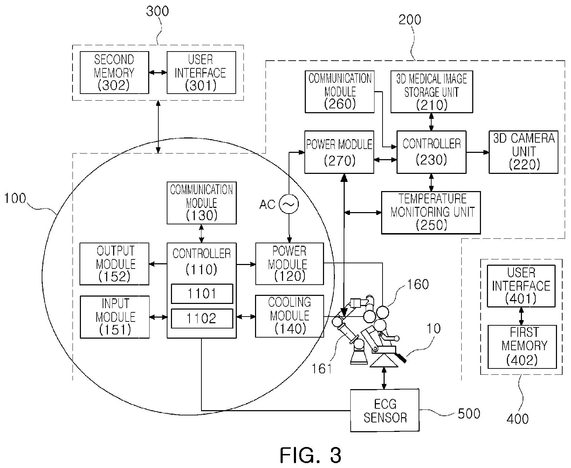

FIG. 3 is an overall block diagram illustrating a brain stimulating device according to an example embodiment of the present disclosure.

As illustrated in FIG. 3, the brain stimulating device may include a magnetic field generating device 100, a navigation device 200, an integrated operator 300, and a cognitive training device 400.

Alternatively, the magnetic field generating device 100 of the brain stimulating device is only an example of stimulating a brain with magnetic field stimulation, and a brain stimulating device according to another example embodiment of the present disclosure may be a brain neurological stimulating device.

The brain neurological stimulating device may be a non-invasive magnetic field stimulating device such as a transcranial magnetic stimulation (TMS) device or a transcranial direct current stimulation (TDCS), or may be a brain neurological stimulating device such as an ultrasound device, or an invasive stimulating device for performing a direct physical stimulation to the brain.

Hereinafter, an embodiment of a magnetic field stimulating device among the brain neurological stimulating devices according to an example embodiment of the present disclosure will be mainly described, which is an exemplary example embodiment and the scope of rights is not limited thereto.

First of all, the magnetic field generating device 100 may dispose a coil 160 on a side in which the user's brain is disposed based on the chair 10 on which the user sits, and may be linked with the coil 160.

The magnetic field generating device 100 according to an example embodiment of the present disclosure is a device that determines a magnetic field output to perform magnetic field stimulation on the user's brain and provides the magnetic field output to the coil 160.

The magnetic field generating device 100 according to an example embodiment of the present disclosure may include a controller 110 determining a corresponding magnetic field in consideration of user data including a motor threshold (MT) and a stimulated brain region, and a power module 120 connected to the controller 110 and the coil 160 for generating a magnetic field, and supplying power so that the coil 160 generates a determined magnetic field.

Furthermore, according to an example embodiment of the present disclosure, the magnetic field generating device 100 may include an output module 152 for displaying an output magnetic field to the user controlling the magnetic field generator 100, a user interface 150 including an input module 151 for manually adjusting the magnetic field output by the user, a communication module 130 communicating with an integrated operator 300 and a brain navigation 200, and a cooling module 140 for monitoring a temperature of the coil 160 and performing cooling when a threshold is exceeded.

Meanwhile, according to an example embodiment of the present disclosure, the cognitive training device 400 may simultaneously or alternately perform cognitive training and brain neurological stimulation in conjunction with the brain neurological stimulating device.

According to an example embodiment, after brain stimulation by the magnetic field generator 100 in conjunction with the magnetic field generator 100, the magnetic field generating apparatus 100 may further include a cognitive training device 400 for performing cognitive training by setting a preset problem corresponding to the brain region so as to activate the stimulated brain region, and may further include an integrated operator 300 communicating with the magnetic field generating device 100 and the cognitive training device 400 and driving the magnetic field generator 100 and the cognitive training device 400 simultaneously or alternately.

The cognitive training device 400 according to an example embodiment of the present disclosure may perform brain stimulation by alternately driving the magnetic field generating device 100, and the cognitive training device 400 may additionally activate the brain region stimulated by the magnetic field generating device 100, or the cognitive training device 400 may complementarily activate the brain region less stimulated by the magnetic field generator 100, thereby improving brain stimulation efficiency, specifically, for example, degenerative brain disease treatment efficiency.

The cognitive training device 400 according to an example embodiment of the present disclosure may include a first memory 402 for storing user treatment data, such as the user's personal information or cognitive training problems or cognitive training scores performed by the user, and a user interface 401 for setting cognitive training problems and allowing the user to feedback answers in response thereto.

The user interface 401 may include various user devices such as a desktop computer, a laptop, a PDA, and a tablet PC, and may be a movable PC to increase user convenience.

Furthermore, the integrated operator 300 according to an example embodiment of the present disclosure is a device that controls a driving time, a time interval, a frequency, and a strength when interworking with the cognitive training device 400 or the magnetic field generating device 100, and simultaneously or alternately driving the cognitive training device 400 and the magnetic field generating device 100.

In preparation for a loss of user data in the first memory 402, the integrated operator 300 may include a second memory 302 for storing a user's treatment history or personal information, and may include a user interface 301 set to allow the users, such as a doctor or a nurse, who can control a magnetic stimulation system, to manually control or check the driving time, the time interval, the frequency, and the intensity.

The second memory 302 may be implemented as a cloud server-type DB as well as an embedded storage device or an external storage device so that the user's treatment history or input user information may be automatically linked to the cloud server and stored. Specifically, the user data may be integrated and stored during remote medical treatment to facilitate data management and increase user convenience.

The user interface 301 may include various user devices such as a desktop computer, a laptop, a PDA, and a tablet PC, and may be a mobile PC to increase user convenience.

Furthermore, the navigation device 200 according to an example embodiment of the present disclosure is a device for detecting the location of the brain region to be stimulated so that the magnetic field generator 100 may be accurately disposed at the position of the user's brain region to be stimulated in conjunction with the magnetic field generating device 100, and providing the brain region position information to the magnetic field generating device 100 so as to guide the position of the magnetic field generating device 100.

The navigation device 200 according to an example embodiment of the present disclosure may include a 3D medical image storage unit 210 in which a 3D medical image is stored, a 3D camera unit 220 for capturing a user sitting on a chair 10 in real time to obtain a camera image, and a controller 230 including a location data calculating unit 231 for calculating location data to dispose the coil 160 by mapping the images obtained from the 3D medical image storage unit 210 and the 3D camera unit 220.

Furthermore, the navigation device 200 may further include a communication module 260 for performing communication in conjunction with the magnetic field generating device 100 and a power module 270 for supplying power to the aforementioned components.

Furthermore, as described in FIG. 3, when the coil 160 is connected to the robot arm 161, the navigation device 200 may further include an RC controller 240 for controlling five-axis driving of the robot arm 161, and a temperature monitoring unit 250 for monitoring a motor temperature of the robot arm 161 to prevent a generated heat from exceeding the safety reference value when heat is generated by a motor operation.

In other words, the bio-navigation robot system that guides the position of the coil 160 corresponding to a region to be stimulated according to another aspect of the present disclosure may include the coil 160 for generating a magnetic field to stimulate the brain region, the robot arm 161 connected to the coil 160 and driven to move the position of the coil 160, the magnetic field generator 100 including a magnetic field conversion module 1101 for controlling the magnetic field output of the coil 160 by adjusting magnetic field-related parameters and a motor threshold measurement module 1102 for measuring the user's motor threshold using the magnetic field conversion module 1101, and the navigation device 200 for controlling the robot arm 161 and moving the coil 160 to a position corresponding to the brain region to be stimulated.

In this case, the coil 160 connected to the robot arm 161 may be connected to one coil, or two or more coils 160 may be connected to one end or a plurality of ends. The coil 160 may be connected differently according to a situation in consideration of a size of a brain region to be stimulated or a treatment target.

Furthermore, before the location data calculating unit 231 calculates the location data, the navigation device 200 may recognize the user's sitting height and adjust the height of the chair 10 seated by the user for treatment. Since the driving range of the robot arm 161 is limited, the height of the user is adjusted to an optimal range for driving. Accordingly, the navigation device 200 may detect the user's sitting height and adjust the height of the chair 10 using a motor so that the user may be disposed within the driving range set for the robot arm 161, thereby optimizing an operation of the robot arm 161 by constantly maintaining a height of the user's head for the robot arm 161.

Specifically, according to an example embodiment of the present disclosure, the navigation device 200 may include an RC controller 240 for receiving location data calculated by the location data calculating unit 231 and controlling a movement direction or a rotation axis of the motor of the robot arm 161 according to the location data, and a temperature monitoring unit 250 for sensing whether the temperature of the robot arm 161 exceeds a predetermined range.

According to an example embodiment, the coil 160 may be moved to a 3D coordinate location calculated for the brain region to be manually stimulated by the user. In order to manually find a location corresponding to the 3D coordinate, the navigation device may further include a laser scanner and a laser pointer. The laser scanner may scan a head position corresponding to the location of the brain region to be stimulated, and the laser pointer may display the position.

Alternatively, as described in FIG. 3, according to an example embodiment of the present disclosure, the robot arm 161 may be driven to a predetermined location according to a location data value calculated by the location data calculating unit 231, and accordingly, the coil 160 may be moved to a location determined by the location data. The location data may include a zero point, a location information point, a location of a brain region to be stimulated, or a location of a motor cortex.

According to an example embodiment of the present disclosure, the robot arm 161 may be driven on a five-axis, and the five-axis may include an X-axis, a Y-axis, a Z-axis and tilting on surfaces formed by each axis. The robot arm 161 may move to a predetermined position and tilt to correspond to the user's head, thus controlling the position of the coil 160 more accurately and enabling user-customized position control. The embodiment is an exemplary embodiment, and a 4-axis operation or a 6-axis operation may be performed, and the claims are not limited to the embodiment. However, the robot arm 161 may be driven on 5 axes or more in order to increase accuracy and precision.

In this case, the RC controller 240 may map the zero point of the robot arm 161 to the zero point of the mapped image, and dispose the robot arm 161 at the mapped zero point before moving the robot arm 161.

Meanwhile, according to another example embodiment, the position of the coil 160 may be controlled using the helmet-type magnetic stimulating unit 163, but this will be described below.

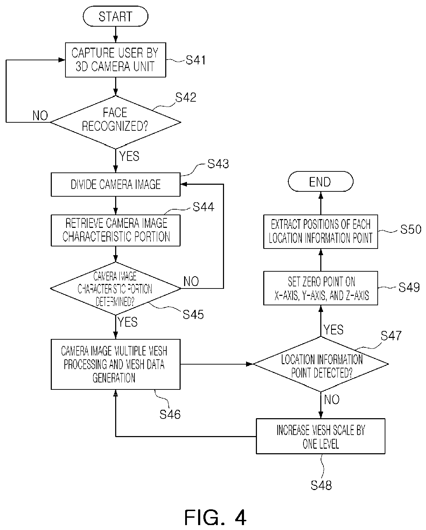

Hereinafter, FIGS. 4 to 8 illustrate flowcharts performed by each module of the location data calculating unit 231, and the location data calculating unit 231 will be described in detail with reference to FIGS. 4 to 8.

FIG. 4 is a flowchart illustrating an image processing method for a camera image of the facial recognition module 2311.

Specifically, as illustrated in FIG. 4, the 3D camera unit 220 captures a user (S41), and if a face is not recognized in a captured camera image (No in S42), the 3D camera unit 220 captures the user again (S41), and if the face is recognized (Yes in S42), the camera image is divided (S43) .

The facial recognition module 2311 according to an example embodiment of the present disclosure may divide the image according to the resolution of the 3D camera image (S43), detect the position of a predetermined face characteristic portion for each of the divided images (S44), and calculate and determine average data of the face characteristic position as a final face characteristic portion of the camera image (S45).

For example, the predetermined face characteristic portion may include a nose, eyes and ears having a depth in the face. When the position of the face characteristic portion in the divided image is detected and an average value of the detected face feature is calculated to determine a camera image face characteristic portion (Yes in S45), the camera image may be subject to a multiple mesh process using the face characteristic portion to generate mesh data (S46) . On the other hand, when the face characteristic portion of the camera image is not determined (No in S45), for example, when the image is excessively shaken to obtain location data far beyond the position of the face characteristic portion, a condition may be changed to return to an operation of dividing the camera image according to the resolution (S43).

That is, according to an example embodiment of the present disclosure, the location data calculating unit 231 may divide the image according to the resolution of the camera image or the medical image and detect the position of the predetermined face protrusion or groove portion for each divided image, may determine an average value of a position of the face protrusion or the face groove portion as a position of a final face protrusion or a final face groove portion, and may generate the mesh data for the camera image or the medical image by considering the final position of the face protrusion or the face groove portion.

The face characteristic portion may vary according to user settings, but in an example embodiment of the present disclosure, the face characteristic portion may denote the face protrusion or the face groove portion, which may refer to an eyebrow bone or a nose protruding from the face, or a recessed eyelid. The 3D camera may detect a depth thereof, and may detect a position or a direction of the entire user's face by detecting the face protrusion or the face groove portion. Furthermore, based on the generated mesh data, the location information point for assisting location detection of the brain region to be stimulated may be detected (S47). The location information point is a predetermined reference point, and may include, for example, a size of a head, a nose, ears, and a forehead, and may include a zero point location to be used as a standard for location coordinates of the brain region to be stimulated.

Alternatively, the mesh data may be generated after detecting the location information point in the camera image. These operations are not bound by a chronological order and may performed simultaneously, or the order before and after the operations may be changed.

When the location information point is detected from the generated mesh data (Yes in S47), a zero point is set on the X-axis, the Y-axis, and the Z-axis accordingly (S49), and locations of each location information point may be extracted based on the zero point (S50). For example, after detecting the location information point on an image from the generated mesh data and setting a zero point having the X-axis, the Y-axis, and the Z-axis accordingly, the zero point may be set as (0,0,0), and (a, b, c) coordinates for each location information point may be obtained.

When the location information point is not detected in the generated mesh data (No in S47), a mesh scale may be increased by one level (S48), and then, the multiplex mesh processing may be performed on the camera image again to generate the mesh data (S46) . This is meant to precisely generate the mesh data by adjusting the mesh data according to the camera image.

Through the aforementioned process, the facial recognition module 2311 may generate the mesh data using the camera image obtained through the 3D camera unit 220 and obtain the location data of the location information point including the zero point.

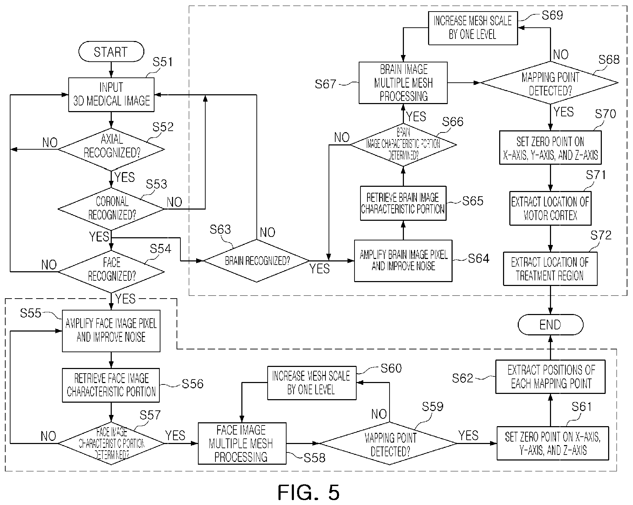

FIG. 5 illustrates an image processing method for a medical image of the medical image analysis module 2312.

Specifically, as illustrated in FIG. 5, a 3D medical image obtained from the 3D medical image storage unit 210 may be received (S51), an axial of the 3D medical image may be recognized (S52), and a coronal may be recognized (S53). The axial refers to an image viewed from the front among 3D medical images, and the coronal refers to a head, which denotes an image viewed from the top.

Accordingly, the axial may be recognized (Yes in S52), and the coronal may be recognized (Yes in S53), thus detecting images of a face region and a brain region at once, or perform image processing by separating the face area from the brain region.

That is, the medical image analysis module 2312 may detect a mapping point or a head in the face region, detect the motor cortex or the location of a brain region to be stimulated in the brain region, and detect them together as a single image.

Alternatively, the face region and the brain region may be detected separately, and in this case, the mesh scale may be changed in consideration of the size of the face region and the brain region, and the mesh data may be generated, thus obtaining more precise mesh data.

In an example embodiment of the present disclosure, as illustrated in FIG. 5, when the face region and the brain region are processed separately, if the user's face region is first recognized in an obtained 3D medical image (Yes in S54), a pixel of a face image of the 3D medical image may be amplified and noise may be improved (S55). Since the medical image may be mixed with noise to lower image quality thereof due to characteristics thereof, this can be improved by the operations described above.

On the other hand, when the axial is not recognized (No in S52), the coronal is not recognized (No in S53), or the user's face region is not recognized in the 3D medical image (No in S54), the 3D medical image may be considered as having an abnormality, and then, the process may return to S51 so as to receive a 3D medical image again.

Next, similarly to the image processing of the camera image of the facial recognition module 2311, the face characteristic portion of the image of the medical image may be retrieved (S56), and an image face characteristic portion of the face region of the 3D medical image among the retrieved face characteristic portions of the image of the face region may be determined (Yes in S57). In this case, the face characteristic portion may refer a face protrusion or a groove portion as described above. The face protrusion or the face groove portion may be determined by detecting 3 points or more.

If the image face characteristic portion of the face region of the 3D medical image is not determined (No in S57), the image quality may be considered poor, and the process may return to S55 so as to amplify the image pixel of the face region of the 3D medical image and remove noise again.

Accordingly, when the face image of the determined 3D medical image is subject to the multiple mesh (S58), and the mapping point is detected from the mesh data obtained therethrough (Yes in S59), the zero point may be set at a preset position on the X-axis, the Y-axis, and the Z-axis (S61), and the position of each mapping point may be extracted based on the zero point (S62). For example, the location data of each mapping point may be obtained by obtaining coordinates (d, e, f) for mapping points of as a nose, a forehead, and ears, based on the zero point (0,0,0) set using a 3D coordinate basis.

Alternatively, as described above, a mapping point may be first detected in the face region image, and then, multiplex mesh data may be generated.

When the location information point is not detected (No in S59), the mesh scale may be increase by one level (S60), and a scale of the mesh data may be adjusted to generate the mesh data again.

On the other hand, the 3D medical image may detect the location information point for a facial appearance, that is, the head including the face, and extract the location data thereof, and may also detect the mapping point for the brain region to be actually stimulated.

That is, the medical image analysis module 2312 according to an example embodiment of the present disclosure may detect the user's face region or the user's brain region of the 3D medical image by recognizing the axial or the coronal of the 3D medical image, and may separately detect the location information point in the face region, the location information point in the brain region, and the position of the motor cortex among the medical images.

In the 3D medical image, in the case in which multi-image scales of the face region and the brain region to be actually stimulated are different from each other and image processing thereof is performed at once, that is, when the mesh data is generated according to a standard of one region thereof, a scale of the other region is not correct, and image quality thereof may be broken, which may not detect an exact mapping point.

Accordingly, according to an example embodiment of the present disclosure, the 3D medical image may classify images into an image of the face region and an image of the brain region, thereby detecting each location information point thereof.

Specifically, as illustrated in FIG. 5, the 3D medical image obtained from the 3D medical image storage unit 210 may be input (S51), the axial of the 3D medical image may be recognized (S52), and the coronal may be recognized (S53).

Accordingly, when the axial is recognized (Yes in S52) and the coronal is recognized (Yes in S53) to recognize the brain region in the obtained 3D (Yes in S63), a pixel of the image of the brain region of the 3D medical image may be amplified and noise may be improved (S64). Since the medical image may be mixed with noise to lower image quality thereof due to characteristics thereof, this can be improved by the operations described above.

On the other hand, when the axial is not recognized (No in S52), the coronal is not recognized (No in S53), or the brain region is not recognized in the 3D medical image (No in S63), the 3D medical image may be considered as having an abnormality in the 3D medical image, and then, the process may return to S 51 so as to receive a 3D medical image again.

Next, similarly to the image processing of the camera image of the facial recognition module 2311, the image characteristic portion of the brain region of the medical image may be retrieved (S65), and a brain image characteristic portion of the 3D medical image among the retrieved characteristic portions of the brain image may be determined (Yes in S66). If the brain image characteristic portion of the 3D medical image is not determined (No in S66), the image quality may be considered poor, and the process may return to S64 so as to amplify the brain image pixel of the 3D medical image and remove noise elimination again. In this case, the characteristic portion refers to a main portion that can determine an overall location of the brain even if the brain moves according to the user's movement, as in the above-described face characteristic portion.

Accordingly, when the image of the brain region of the determined 3D medical image is subject to the multiple mesh (S67), and the mapping point is detected from the mesh data obtained therethrough (Yes in S68), the zero point may be set at a preset position on the X-axis, the Y-axis, and the Z- axis (S70), and the position of the motor cortex or the brain region to be stimulated, which determines the motor threshold (MT), may also be detected based on the brain region set to the zero point. Accordingly, the position of the motor cortex may be extracted based on the zero point (S71), and the location of a brain treatment region to be stimulated may be extracted (S72).

For example, the location data of the motor cortex may be obtained by obtaining coordinates (g, h, i) for the motor cortex based on the zero point (0, 0, 0) set using a 3D coordinate basis.

If the mapping point is not detected (No in S68), the mesh scale may be increased by one level (S69), and the mesh data may be generated again by adjusting the scale of the mesh data.

Accordingly, the medical image analysis module 2312 may extract a face image and a brain image from the 3D medical image, respectively, and detect the mapping point based on the extracted images.

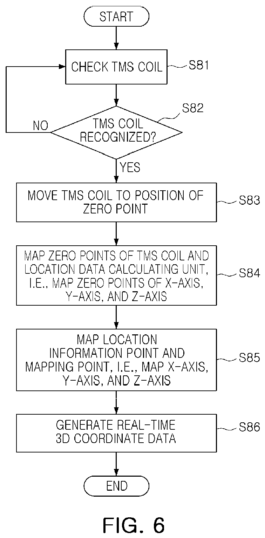

FIG. 6 is a flowchart illustrating an operation of the image mapping module 2312 according to an example embodiment of the present disclosure.

The image mapping module 2313 according to an example embodiment of the present disclosure may generate real-time 3D coordinate data by mapping at least two or more location information points of the coil 160 for performing the magnetic stimulation, the facial recognition module 2311, and the medical image analysis module 2312, or zero coordinates.

For example, the coil 160 may include a TMS coil, and hereinafter, the coil 160 will be described as the TMS coil in FIG. 6, but the present disclosure is not limited to the coil.

Specifically, as illustrated in FIG. 6, the image mapping module 160 may check the TMS coil (S81), and may recognize the TMS coil (S82). The image mapping module 160 may check whether the TMS coil of the magnetic field generating device 100 is connected or powered through the communication module 260.

When the image mapping module 160 recognizes the TMS coil (Yes in S82), the TMS coil is moved to a position of the zero point (S83). Before the TMS coil is moved to a predetermined position, the TMS coil is moved to the position of the zero point so that it is moved to a more accurate coordinate.

Next, the zero point of the TMS coil and the zero point of the location data calculating unit 231, that is, the zero points of the X-axis, the Y-axis, and the Z-axis set in the mapped image obtained by the facial recognition module 231 and the medical image analysis module 2312 may be mapped (S84). The zero point set by the TMS coil, the zero point in the camera image obtained by the facial recognition module 2311 through the 3D camera unit 220, and the zero points in the face image and the brain image of the 3D medical image obtained by the medical image analysis module 2312 through the 3D medical image storage unit 210 may be mapped.

The camera image or the 3D medical image that has been obtained and subject to image processing, respectively, is mapped by means of the zero point to integrate a medical image of an actual user with a medical image with a camera image of a user who currently moves in real time, and the coil 160 moving to the brain region to be actually stimulated also performs the zero-point mapping to synchronize the obtained image with the coil 160.

When the zero-point mapping of the X-axis, the Y-axis, and the Z-axis is completed, the location information point of the facial recognition module 2311 and the mapping point of the medical image analysis module 2312 may be mapped (S85) to complete image integration, and real-time 3D coordinate data thereof may be generated (S86) so that the TMS coil may move to a calculated 3D coordinate position.

According to an example embodiment of the present disclosure, the position of the robot arm 161 may be more accurately controlled by disposing the robot arm 161 at a mapped zero point before moving to the calculated 3D coordinate position.

On the other hand, as described in FIG. 6, the image mapping module 2313 may generate real-time 3D coordinate data and transmit the calculated 3D coordinate data and the location data of the motor cortex to the motor threshold control module 232 to assist in a motor threshold measurement.

The motor threshold control module 232 may allow the RC controller 240 to move the robot arm 161 to the 3D coordinate of the motor cortex using the calculated 3D coordinate data and the location data of the motor cortex, and may allow the motor threshold measurement module 1102 to measure the motor threshold.

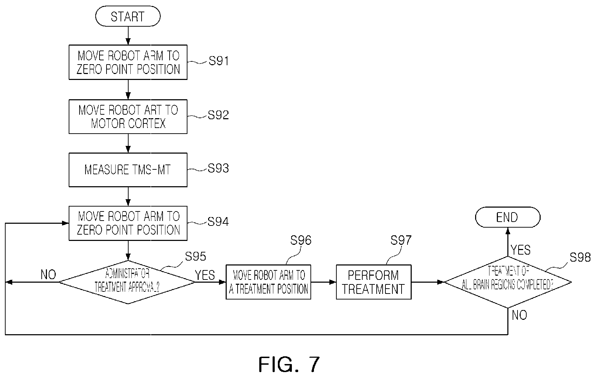

FIG. 7 illustrates a flowchart in which a navigation device according to an example embodiment of the present disclosure guides the position of the robot arm 161, and as described in FIG. 7, the robot arm 161 may be moved to a zero point position (S91) and then the robot arm 161 may be moved to the motor cortex (S92).

When the robot arm 161 moves to the motor cortex and the coil 160 may perform magnetic field stimulation on the motor cortex, the motor threshold measurement module 1102 may measure a TMS-MT (S93). The motor threshold of the user may be measured therethrough.

To receive a magnetic stimulation method, while the user is in a fixed state without a motion, his/her brain needs to be stimulated, and accordingly, an output of the magnetic field has to be adjusted to a level equal to or less than the motor threshold (MT) in which fingers and the like move, while the output of the magnetic field needs to remain close to the motor threshold to increase the stimulation efficiency of the brain.

The motor threshold may vary depending on the user's race, gender, and the like, but also depend on the user's condition, and for accurate and safe treatment, it is very necessary to accurately confirm the motor threshold just before treatment.

Accordingly, the motor threshold measurement module 1102 may measure the motor threshold by gradually adjusting the magnetic field strength by the magnetic field conversion module 1101, and may determine a magnetic field parameter to be performed for brain stimulation according to the motor threshold.

If there is no finger response or motor threshold measurement sensor such as an ECG sensor or an EMG sensor while passing through the aforementioned process in the motor cortex, it may be determined that the motor cortex is not properly stimulated, and the RC controller 240 may be driven such that the robot arm 161 may perform a position search in a circle around the location data obtained as the motor cortex. The robot arm 161 may move the coil 160 in a circle to detect the motor cortex location and store the location thereof. Furthermore, the detected motor cortex location data may be learned from an artificial intelligence module to detect the motor cortex location more accurately and quickly, thus obtaining the motor threshold.

When the motor threshold measurement is completed, the robot arm 161 is moved back to the zero point position (S94), and when an administrator approves the treatment (Yes in S95), the robot arm 161 may be moved to the treatment position (S96). This is meant to increase treatment efficiency by more accurately controlling the position of the robot arm 161. When the administrator's treatment approval is not obtained (No in S95), the robot arm 161 may continue to wait at the zero point position.

The robot arm 161 may be moved to the treatment position, and the coil 160 may generate a magnetic field to perform brain stimulation treatment (S97).

When performing the brain stimulation, a total of six brain regions are stimulated: the Broca region, the Werniche region, the left and right dorsolateral prefrontal cortex (DLPFC) regions, and the left and right parietal somatosensory association cortex (PSAC) regions.

If at least two or more of the plurality of brain regions are set to be stimulated, after performing the treatment (S97), it is determined whether all brain regions are treated (S98), and when all brain regions have been treated (Yes in S98), the treatment may be terminated, and when all brain regions are not treated (No in S98), the process may return to S94 to move the robot arm to the zero point position, and operation S95 to S98 may be repeated.

Through the above-described configuration, there is no need for a separate assistant other than the user to help the coil 160 be disposed in the brain region to be stimulated, and the position of the coil 160 may be automatically controlled according to the user's movement, and accordingly, the user may perform the magnetic field stimulation alone, thereby reducing costs by reducing time and manpower, and a user-customized search may be made possible by more accurate control of the position of the coil 160, thereby increasing treatment efficiency.

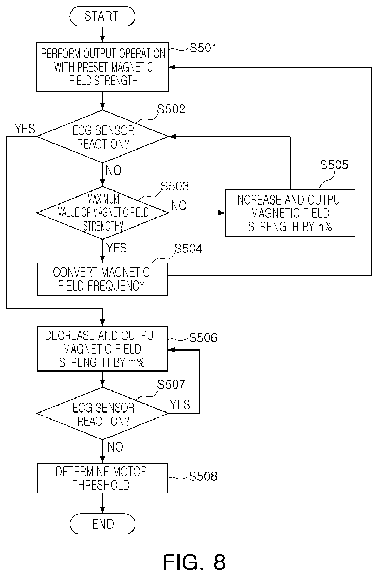

Meanwhile, FIG. 8 is a flowchart illustrating a motor threshold measurement module 1102 according to an example embodiment of the present disclosure.

The motor threshold measurement module 1102 may stimulate a motor cortex region in which the coil 160 is responsible for the motor region of the brain, and may then detect a change in a movement of a hand or a pulse to determine whether stimulation is transmitted to the brain.

Specifically, the motor thresholds of the users are different from each other for each person, and the same user has different motor thresholds for each day and for each condition. In particular, Asians have much higher motor thresholds than Westerners, and in a case in which there is no control method for accurate motor threshold measurement, when there is no finger movement at the randomly provided magnetic field output, or an ECG sensor 500 does not recognize a change in pulse or an EMG sensor does not detect a change in an action potential of a muscle or a nerve, the user was no longer able to receive the magnetic stimulating treatment.

Accordingly, the motor threshold measurement module 1102 according to an example embodiment of the present disclosure may measure the user's motor threshold by gradually adjusting the magnetic field strength by the magnetic field conversion module 1101, or when the magnetic field strength reaches a maximum output of the magnetic field, the motor threshold measurement module 1102 may measure the user's motor threshold by increasing a magnetic field frequency by a predetermined ratio and then gradually adjusting the magnetic field strength.

Furthermore, the magnetic field generating device according to an example embodiment of the present disclosure may include a motor threshold measurement sensor such as an ECG sensor 500 or an EMG sensor for sensing the user's ECG to detect an ECG change when the magnetic field of the coil 160 generated by the magnetic field conversion module 1101 exceeds the motor threshold.

Hereinafter, according to an example embodiment of the present disclosure, the position of the coil 160 may be automatically controlled using the robot arm 161, and since there is no separate manpower to check the movement of fingers, an example embodiment of measuring the motor threshold using the ECG sensor 500 will be described below. Even when a helmet-type magnetic stimulating unit 163 described below is used, the motor threshold may be measured using the ECG sensor 500.

As illustrated in FIG. 8, the MT measurement module 1102 according to an example embodiment of the present disclosure may perform first an output operation with a preset magnetic field strength (S501). For example, the MT measurement module 1102 may set a possible magnetic field strength, or a moderate magnetic field strength or magnetic field frequency in a magnetic field frequency range, and may output a magnetic field. A standard point is set to measure the user's motor threshold.

Then, in a first stage, the ECG sensor 500 cannot measure the change in pulse, and there is no sensor response (No in S502), and when the magnetic field strength is not a maximum value (No in S503), the magnetic field frequency may be fixed, and the magnetic field strength may be adjusted by increasing by a predetermined ratio (n%) (S505).

The magnetic field strength may be increased until the ECG sensor 500 measures a change in pulse. In this case, when a response is detected in the ECG sensor (Yes S502), it is determined that the motor cortex of the user's brain exceeds a reacted motor threshold, and the magnetic field strength may be reduced and output at a predetermined ratio m% set to be less than the predetermined ratio n% (S506).

When the ECG sensor reacts (Yes in S507), it is determined that the motor cortex has not yet reached a threshold just before the motor threshold, and the process may return to S506 to lower and output the magnetic field strength. When the ECG sensor does not react (No in S507), the threshold just before the motor threshold may be detected. Accordingly, the motor threshold may be determined (S508), and the threshold of the motor threshold becomes a reference value of the magnetic field strength to be used for brain stimulation.

On the other hand, if the ECG sensor fails to measure the change in pulse even when the magnetic field strength increases to reach a maximum value of the magnetic field strength (Yes in S503), a magnetic field frequency may be improved to perform magnetic field frequency conversion of improving a magnetic field output (S504).

After increasing the magnetic field frequency by a predetermined ratio, the magnetic field strength may return to an initial level and output a medium-level magnetic field strength (S501), and when the response of the ECG sensor is not detected, the magnetic field strength may be increased again by a predetermined ratio (n%) until the ECG sensor measures the change in pulse.

In this case, according to an example embodiment of the present disclosure, when the magnetic field frequency can no longer be improved by converting the magnetic field frequency in S504, it is determined that exact magnetic field stimulation has not been delivered to the motor cortex, and the motor cortex may be detected in a circle around a point in which the coil 160 is currently disposed. A newly searched motor cortex location may be stored in a database or a server, and the artificial intelligence module may be trained with the data.

That is, the brain stimulating device according to an example embodiment of the present disclosure may further include a motor threshold control module 232 for measuring the motor threshold of the user by adjusting the magnetic field strength or frequency of the coil 160 after disposing the coil 160 at a head position corresponding to the motor cortex position in the brain region, or determining the coil 160 disposed at a head position corresponding to the motor cortex position.

This allows all users to detect motor thresholds (MT) that suit themselves, and the threshold just before the motor threshold may be set to a standard value of the magnetic field strength to be used for brain stimulation, and accordingly, the lack of mobility may control the user not to move while performing magnetic stimulation, and at the same time, the magnetic stimulation may be performed with a maximum magnetic field output to perform the brain stimulation, thereby maximizing treatment efficiency with safety secured.

Although the ECG sensor 500 has been described above, this is only an exemplary embodiment, and the present disclosure may include all motor threshold measurement sensors that can detect the action potential of muscles or nerves to check the motor threshold response.

In the above-described example embodiment, the position of the coil 160 is adjusted by connecting the coil 160 to the robot arm 161 to drive the robot arm 161.

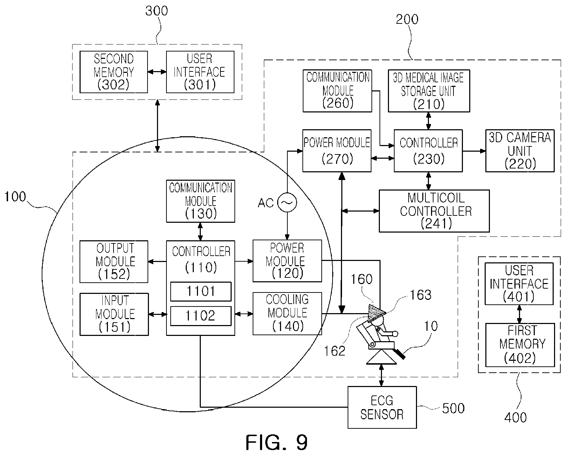

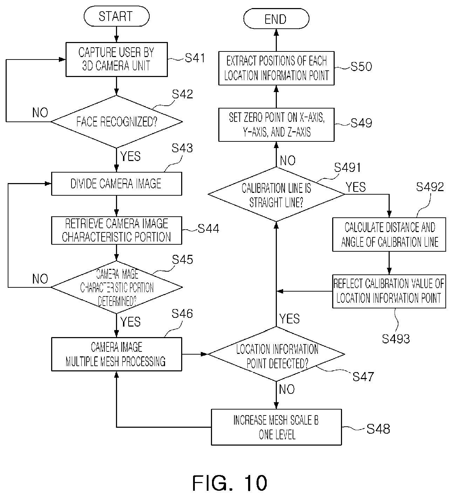

Hereinafter, FIG. 9 illustrates an overall block diagram of a brain stimulating device according to another example embodiment of the present disclosure, and FIGS. 10 to 11 illustrate an embodiment in which the coil 161 is disposed in a helmet-type magnetic stimulating unit 162, and the coil 160 generates a magnetic field at the brain region position to be stimulated. The same configuration as the above-described content will be omitted to avoid duplicate content.

As illustrated in FIG. 9, a brain stimulating device for guiding a position of a coil 160 corresponding to a region to be simulated, according to another example embodiment of the present disclosure, may include a plurality of coils 160 for stimulating a brain region by generating a magnetic field, a helmet-type magnetic stimulating unit 162 in which the plurality of coils 160 are densely disposed in a helmet-type fixing portion worn by a user on his/her head, a magnetic field generating device 100 including a magnetic field conversion module 1101 for controlling a magnetic field output of the coil 160 by adjusting a magnetic field-related parameter and a motor threshold measurement module 1102 for measuring the user's motor threshold using the magnetic field conversion module 1101, and a navigation device 200 for calculating location data of the brain region to be stimulated and determining the coil 160 disposed at a position corresponding to the brain region to be stimulated. The navigation device 200 may further include a multicoil controller 241 for selecting at least one of the plurality of coils 160 according to the location data calculated by a location data calculating unit 231 and transmitting an identification mark of the selected coil 160 to the magnetic field generating device 100.

Furthermore, according to another example embodiment, the helmet-type magnetic stimulating unit 162 includes a calibration bar 163 horizontally disposed in a lower end of the fixing portion. The navigation device may obtain a tilted angle of the calibration bar 163 or a distance to a location information point of the user's camera image using the 3D camera unit 220, may calibrate the location information point in consideration of calibration data obtained by calculating the angle or the distance of the calibration bar 163, and may transmit at least one identification mark of the plurality of coils 160 disposed corresponding to the brain region to be stimulated based on a calibrated location information point, to the magnetic field generating device 100.

Specifically, the helmet-type magnetic stimulating unit 162 may be in close contact with a scalp according to the user's head, and may be worn after attaching hair to the scalp through a separate rubber cap. The plurality of coils 160 are densely disposed in the helmet-type magnetic stimulating unit 162, and when at least one of the plurality of coils 160 is driven, a magnetic field may be generated and the brain region in a position corresponding to the coil 160 may be stimulated.

In addition, according to an example embodiment of the present disclosure, the coils 160 may be disposed in the form of a circular coil, respectively, or in the form of an eight-shaped coil, or the coils 160 may be connected and disposed so that each coil operates in a pair. In this case, the coils 160 operating in the pair may be connected to overlap between the coils 160.

For example, when the coil 160 is an eight-shaped TMS coil, a sharp waveform magnetic field may be generated at a bonding portion in which two circles are bonded in an eight-shape, and the plurality of coils 160 may be disposed so that a magnetic field generated from the bonding portion may magnetically stimulate a corresponding brain region.

Furthermore, the multicoil controller 241 according to an example embodiment of the present disclosure may control a switching operation of the switch connected to each coil 160 to control on or off of all or a portion of the coil 160. For example, after all coils 160 are switched off, only a selected coil may be switched and operated.

Furthermore, the helmet-type magnetic stimulating unit 162 is a component in which the coil 160 disposed in a predetermined position stimulates a corresponding brain region, and when the helmet-type magnetic stimulating unit 162 is worn crookedly or incorrectly, other brain regions may be stimulated. Accordingly, in order to supplement and correct the problem, the calibration bar 163 may be disposed along the head at the lower end of the helmet-type magnetic stimulating unit 162.

That is, when the 3D camera unit 220 takes an image of the user wearing the helmet-type magnetic stimulating unit 162, the calibration bar 163 may be captured horizontally, and when the calibration bar 163 is captured slopingly because the helmet-type magnetic stimulating unit 162 is incorrectly worn, this may be reflected to calibrate the coordinates of the location information point.

Specifically, for example, as described in FIG. 10, when the facial recognition module 2311 obtaining the camera image captured by the 3D camera unit 220 detects the location information point by processing the image (Yes in S47), an additional calibration operation may be performed.

In the camera image or the mesh data generated from the camera image, when a calibration line formed by the calibration bar 163 is not a straight line (No in S491), it may be seen that the calibration bar 163 is tilted.

Accordingly, an angle in which the calibration line formed by the calibration bar 163 is formed with the horizontal line, and a distance between the calibration line and the location information point may be calculated (S492), and a calibration value may be reflected in the location information point by reflecting a degree to which the calibration line is tilted (S493), thereby obtaining the location data of the location information point.

Specifically, by comparing a straight line formed by the location information point and a straight line formed by the calibration bar 163, it may be detected whether a distance difference between the straight lines remains constant or the distance difference changes, thereby detecting whether the calibration bar 163 is tilted.

Furthermore, zero points of the X-axis, the Y-axis, and the Z-axis may be set (S49), and locations of each location information point may be extracted based on the zero points (S50). Here, the zero points of the X-axis, the Y-axis, and the Z-axis and each location information point may be location data reflecting the calibration value calculated above.

For example, when the calibration bar 163 is twisted and the location set to the zero point is set to coordinates of (x, y, z), a current location of the preset zero point may be calibrated to (0, 0, 0) by reflecting the calibration value, and accordingly, the location information point (a, b, c) may be calibrated to (a', b', c').

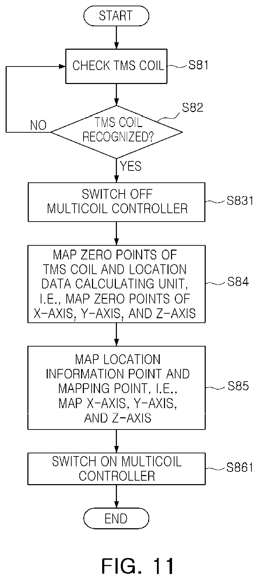

Furthermore, as illustrated in FIG. 11, when a TMS coil check and recognition is completed (Yes in S81 and S82), the image mapping module 2313 switches off the multicoil controller 241 (S831). The multicoil controller 241 switches off all switches connected to the coil 160.

Furthermore, the zero points of the X-axis, the Y-axis, and the Z-axis of the TMS coil, the facial recognition module 2311, and the medical image analysis module 2312 may be mapped (S84). Here, the zero point of the TMS coil and the zero point of the facial recognition module 2311 may be zero points reflecting the calibration value using the calibration bar 163.

When the zero-point mapping is completed, the location information points of the facial recognition module 2311 and the medical image analysis module 2312 may be mapped (S85). Here, the location information point of the facial recognition module 2311 may be a zero point reflecting the calibration value using the calibration bar 163.

When integration of the image is completed, the multicoil controller 241 may be switched on (S861), and at least one of the coils 160 disposed in the helmet-type magnetic stimulating unit 162 at a location corresponding to the location information point may be controlled to generate a magnetic field. In this case, the multicoil controller 241 may switched on only the selected coil 160.

According to an example embodiment, the multicoil controller 241 may select the coil 160 to be switched on differently depending on the treatment method, and may select and switched on one coil or a plurality of coils. Furthermore, the multicoil controller 241 may control one or more coils 160 to change at least one of a frequency, a period, and a pulse shape at the same time or with a time difference.

Accordingly, the location data calculating unit 231 may calculate location data including real-time 3D coordinates reflecting the calibration value, and transmit the location data to the motor threshold control module 232, so that the coil 160 disposed at a location corresponding to the motor cortex may be controlled to generate the magnetic field in order to measure the motor threshold, or the location data calculating unit 231 may transmit the location data to the multicoil controller 241 to determine the position of each coil 160 corresponding to the brain region to be stimulated.

Meanwhile, according to another example embodiment of the present disclosure, a brain stimulating device may include a plurality of coils 160 for stimulating a brain region by generating a magnetic field, a helmet-type magnetic stimulating unit 162 in which the plurality of coils 160 are densely disposed in a helmet-type fixing portion worn by a user on his/her head, a magnetic field generating device 100 including a magnetic field conversion module 1101 for controlling a magnetic field output of the coil 160 by adjusting a magnetic field-related parameter and a motor threshold measurement module 1102 for measuring the user's motor threshold using the magnetic field conversion module 1101, and a navigation device 200 for calculating location data of the brain region to be stimulated and determining the coil 160 disposed at a position corresponding to the brain region to be stimulated. The navigation device 200 may further include a multicoil controller 241 for transmitting an identification mark of at least one of the plurality of coils 160 according to the location data calculated by a location data calculating unit 231 to the magnetic field generating device 100.

In this case, at least one of a gyro sensor or an acceleration sensor connected to the helmet-type magnetic stimulating unit 162 may be used to sense an occurrence of a tilt of the helmet-type magnetic stimulating unit 162, thus calibrating the location information point of the camera image in consideration of calibration data obtained by calculating a tilted angle of the helmet-type magnetic stimulating unit 162, and the identification mark of at least one of the plurality of coils 160 disposed corresponding to the brain region to be stimulated based on the calibrated location information point may be transmitted to the magnetic field generating device 100.

In other words, according to another example embodiment of the present disclosure, a helmet-type magnetic stimulating unit 162 to which the calibration bar 163 is not connected may be included.

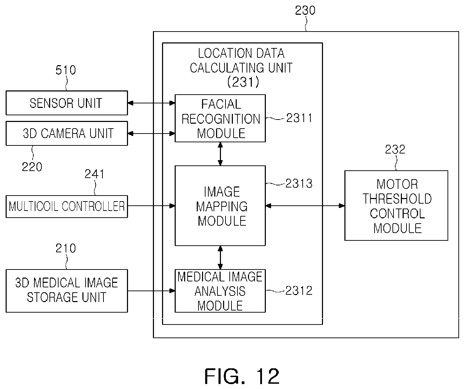

As illustrated in FIG. 12, a sensor unit 510 may be disposed in the helmet-type magnetic stimulating unit 162 instead of the calibration bar 163, and the sensor unit 510 may include a gyro sensor or an acceleration sensor. The sensor unit 510 may be provided with one sensor or a plurality of sensors.

According to another example embodiment of the present disclosure, the facial recognition module 2311 may sense an occurrence of a tilt from the sensor unit 510. Specifically, after detecting the location information point (S47), zero points of the X-axis, the Y-axis, and the Z-axis are set (S49) and before extracting locations of each location information point (S50), when the sensor unit 510 senses that the helmet-type magnetic stimulating unit 162 is tilted, the calibration value may be reflected in the location information point using the tilted angle obtained from the sensor unit 510.

Instead of using the 3D camera unit 220 to reflect the calibration value, the sensor unit 510 may be utilized to sense whether the helmet-type magnetic stimulating unit 162 is accurately worn and may perform the calibration.

Alternatively, both the calibration bar 163 and the sensor unit 510 may be disposed on the helmet-type magnetic stimulating unit 162. The 3D camera unit 220 may be used to calculate an angle at which a calibration line of the calibration bar 163 is tilted, and a distance to each location information point, and obtain a calibrated location information point of 1 reflecting the angle and the distance, and the gyro sensor or the acceleration sensor of the sensor unit 510 may be used to calculate a tilted angle, and obtain a corrected position information point of 2 reflecting the tilted angle.

Furthermore, by optimizing the location information point of 1 and the location information point of 2, a final location information point and a zero point reflecting the optimized points may be extracted. Location data of the location information point may be more accurately obtained by increasing parameters to be considered.

Furthermore, according to an example embodiment of the present disclosure, if the final location information point value obtained by reflecting the calibration value differs from the location information point obtained using the AI from a big data server, a coefficient for optimizing the location information point may be modified and a process of calculating the location information point may be performed again.

Through the aforementioned process, it is possible to retrieve user-customized location information points by considering different heads or different brain shapes and different distances for each user, and to accurately detect the location information points in a shorter period of time, thereby reducing costs and time as well as further increasing treatment efficiency.

Furthermore, a navigation device using the robot arm 162 according to one example embodiment of the present disclosure may be used as a hospital device to reduce manpower and save time, and a navigation device using the helmet-type magnetic stimulating unit 163 according to another example embodiment of the present disclosure may be used as a home device. The magnetic field stimulation may be performed without separate medical personnel, and magnetic field stimulation results may be stored through the server so that medical personnel may remotely manage the magnetic field stimulation.

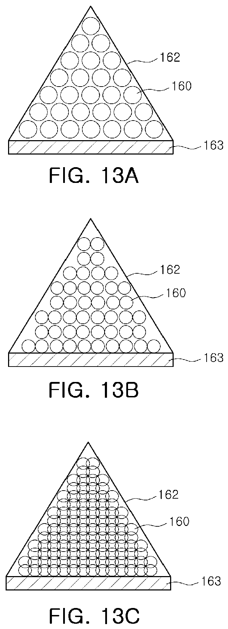

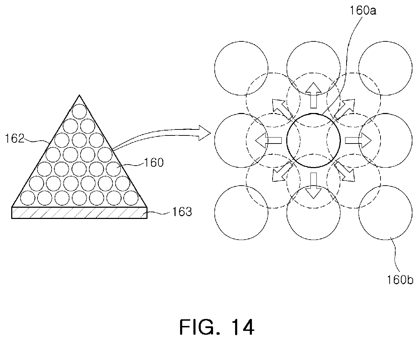

On the other hand, FIG. 13 is a view schematically illustrating an arrangement of a coil 160 disposed in a helmet-type magnetic stimulating unit 162 according to another example embodiment of the present disclosure, and FIG. 14 is a view illustrating a structure of a coil 160 in a helmet-type magnetic stimulating unit 162 according to another example embodiment of the present disclosure.

Although FIG. 13 illustrates a triangular shape, the helmet-type magnetic stimulating unit 162 may be worn on the user's head and may include any form that may surround the brain region, and the shape is not limited by the exemplary embodiment.

Furthermore, the helmet-type magnetic stimulating unit 162 may connect the calibration bar 163 at a lower end thereof, and may detect whether the user wears the helmet-type magnetic stimulating unit 162 in a correct position, by detecting that the calibration bar 163 is tilted or twisted, and provide information to perform the calibration by the titled angle.

According to an example embodiment, as illustrated in FIG. 13A, the coil 160 may be arranged a form in which one coil is disposed at regular intervals in the helmet-type magnetic stimulating unit 162. The regular intervals may be variously adjusted, but each coil may be arranged densely enough to stimulate the brain region.

Furthermore, according to an example embodiment, as illustrated in FIG. 13B, the coil 160 may be arranged in a form in which eight-shaped coils are disposed at regular intervals. The eight-shaped coil may accurately stimulate only a point in which the magnetic field is sharply formed at a part in which the two circles contact each other to perform the magnetic field stimulation. The eight-shaped coils may be densely disposed in the helmet-type magnetic stimulating unit 162.

In this case, although FIG. 13B illustrates that the eight-shaped coils are placed and coils are not disposed on an external portion of the helmet-type magnetic stimulating unit 162, this illustrates a cross-section of the helmet-type magnetic stimulating unit 162, and an actual helmet-type magnetic stimulating unit 162 may be configured in a three-dimensional form in which the eight-shape coils may be turned and tightly arranged even on the external portion thereof.

Furthermore, according to another example embodiment, as illustrated in FIG. 13^{©}, each of coils 160 may be disposed to overlap each other. As illustrated in FIG. 13B, use of the eight-shaped coils may efficiently stimulate only a specific position because electromagnetic force is sharply formed at a contact point in which two coils are in contact with each other, and as illustrated in FIG. 13C, use of the overlapped coil structure may increase an area affected by the magnetic field, thereby increasing the electromagnetic force that stimulates the brain in unit coils.

FIG. 13C also illustrates that the coil is not disposed on the external portion of the helmet-type magnetic stimulating unit 162, but this illustrates the cross-section of the helmet-type magnetic stimulating unit 162, and actual helmet-type magnetic stimulating unit 162 may be configured in a three-dimensional form in which the eight-shape coils may be turned and tightly arranged even on the external portion thereof.

As described in FIG. 13, from a time at which the coil 160 is first disposed in the helmet-type magnetic stimulating unit 162, the coils 160 may be disposed at regular intervals, the eight-shaped coils formed in pairs may be disposed, or the coils 160 may be disposed to overlap each other, but an arrangement of the coils 160 may be changed according to the situation through the helmet coil structure illustrated in FIG. 14.

That is, a motor may be connected to at least one of coils 160a disposed in the helmet-type magnetic stimulating unit 162, and the multicoil controller 241 may control the motor so that the coils 160a connected to the motor overlaps at least one of peripheral coils 160b, and may determine whether the coil 160a connected to the motor is moved, a direction of the movement, and a distance of the movement.

The peripheral coil 160b refers to a coil which is not connected to the motor and is disposed around the coils 160a so that the coil 160a connected to the motor can get close. In an example embodiment illustrated in FIG. 14, nine coils are assumed to be one set, in which eight coils disposed vertically, horizontally and diagonally disposed based on one coil 160a disposed at a center refer to peripheral coils 160b.

As illustrated in FIG. 14, the coils 160 may be disposed at regular intervals in the helmet-type magnetic stimulating unit 162 (illustrated as a solid line), and the coil 160 may include a coil 160a in which the motor is mounted on at least one of the disposed coils 160, and a coil 160b fixed to a position disposed without the motor (the coil 160a is illustrated as a thick solid line, and the coil 160b is illustrated as a thin solid line) . The coil 160a equipped with the motor may move vertically, horizontally and diagonally, as indicated by an arrow, and may move toward fixed coils 160b and create a contact point to form a pair of coils, or may move to overlap each other.

When the coil 160 is fixed in a specific position, it may be difficult to change the position of the coil 160 even if the brain is not accurately stimulated. For example, in a state of accurately wearing the helmet-type magnetic stimulating unit 162 through the calibration bar 163, in a case in which stimulation to the upper part of the brain is required, when the coil 160a equipped with the motor is moved upward to overlap the coil 160b, an area affected by the magnetic field with respect to an upper region may be increased to adjust a position or an area of the brain region to be stimulated.

For another example, when the brain region to be stimulated is disposed in a lower diagonal direction, the coil 160a equipped with the motor may be moved diagonally downward to overlap the coil 160b, thereby adjusting the position and the area thereof so that the magnetic field reaches the corresponding region.

In FIG. 14, the motor is mounted on the coil 160a disposed at the center by setting nine coils 160 as one set, but this is only an exemplary embodiment and may be changed or replaced within the range that may be clearly derived by a person skilled in the art.

In other words, the coil 160 may select various coils as one circular coil or an eight-shaped coil, the arrangement of the coils 160 may also be selected in various arrangement forms, such as an arrangement of the coils at regular intervals, a dense arrangement of the coils without intervals, or an arrangement of connecting and overlapping the coils, and the motor may be mounted on at least one coil to move the position of the coil. This may be determined in consideration of a region for treatment such as the brain region to be stimulated, a treatment target, or the like.

The present disclosure is not limited to the embodiment described above and the accompanying drawings. The scope of rights of the present disclosure is intended to be limited by the appended claims. It will be understood by those skilled in the art that various substitutions, modification and changes in form and details may be made therein without departing from the spirit and scope of the present disclosure as defined by the appended claims.

### DESCRIPTION OF REFERENCE CHARACTERS

100: MAGNETIC FIELD GENERATING DEVICE 110: CONTROLLER
1101: MAGNETIC FIELD CONVERSION MODULE 1102: MOTOR THRESHOLD MEASUREMENT MODULE
120: POWER MODULE 130: COMMUNICATION MODULE
140: COOLING MODULE 150: USER INTERFACE
151: INPUT MODULE 152: OUTPUT MODULE
160: COIL 161: ROBOT ARM
162: HELMET-TYPE MAGNETIC STIMULATING UNIT 163: CALIBRATION BAR
200: NAVIGATION DEVICE 210: 3D MEDICAL IMAGE STORAGE UNIT
220: 3D CAMERA UNIT 230: CONTROLLER
231: LOCATION DATA CALCULATING UNIT 2311: FACIAL RECOGNITION MODULE
2312: MEDICAL IMAGE ANALYSIS MODULE 2313: IMAGE MAPPING MODULE
232: MOTOR THRESHOLD CONTROL MODULE 240: RC CONTROLLER
241: MULTICOIL CONTROLLER 250: TEMPERATURE MONITORING UNIT
260: COMMUNICATION MODULE 270: POWER MODULE
300: INTEGRATED OPERATOR 301: USER INTERFACE
302: SECOND MEMORY 400: COGNITIVE TRAINING DEVICE
401: USER INTERFACE 402: FIRST MEMORY
500: ECG SENSOR 510: SENSOR UNIT

## Claims

1. A brain stimulating device including a navigation device for guiding a position of a coil, wherein the navigation device comprises:
a 3D camera unit for capturing an image of a user to obtain a 3D camera image; and
a location data calculating unit for mapping the camera image to a 3D medical image including brain region information or mapping the 3D medical image to the camera image to obtain a mapped image, and calculating real-time 3D coordinates as location data of a brain region to be stimulated from the mapped image,
wherein the location data calculating unit includes a medical image analysis module for detecting a motor cortex and a location of a brain region to be stimulated in the brain region of the medical image, calculates real-time 3D coordinates for the motor cortex and the location of the brain region to be stimulated from the mapped image, and provides the 3D coordinates to a magnetic field generating device so that magnetic stimulation may be performed at a magnetic field strength determined by a motor threshold measured from the coordinates of the calculated motor cortex.

2. The brain stimulating device including a navigation device for guiding a position of a coil of claim 1, wherein the location data calculating unit calibrates a preset zero point in the camera image or the medical image based on the mapped image, and generates real-time 3D coordinates in the mapped image based on a calibrated zero point,

3. The brain stimulating device including a navigation device for guiding a position of a coil of claim 1, wherein the location data calculating unit is configured to,
divide an image according to a resolution of the camera image or the medical image, and detect a position of a preset face protrusion or a preset face groove portion for each of the divided images, and
determine an average value of a position of the face protrusion or the face groove portion as a position a final face protrusion or a final face groove portion, and generate mesh data for the camera image or the medical image in consideration of the final face protrusion or the final face groove portion.

4. The brain stimulating device including a navigation device for guiding a position of a coil of claim 1, wherein the location data calculating unit includes an artificial intelligence model learned through artificial intelligence using data from a server, and
the location data calculating unit matches, to user information, at least one data among a location information point in the camera image varying depending on a user, a mapping point in the medical image varying depending on the user and a matching point between the location information point and the mapping point to store the data in the server, or stores learning results of the location data calculating unit in the server.

5. The brain stimulating device including a navigation device for guiding a position of a coil of claim 4, further comprising:
a cloud server for receiving and storing the data stored in the server.

6. The brain stimulating device including a navigation device for guiding a position of a coil of claim 4, wherein the location data calculating unit extracts at least one of the location information point in the camera image, the mapping point in the medical image, and the matching point between the location information point and the mapping point using the artificial intelligence model.

7. The brain stimulating device including a navigation device for guiding a position of a coil of claim 1, further comprising:
a magnetic field generating device including: a coil for stimulating a brain region of a user by generating a magnetic field; a robot arm connected to the coil and driven to move the position of the coil; a magnetic field conversion module for controlling a magnetic field output of the coil by adjusting a magnetic field-related parameter; and a motor threshold measurement module for measuring a motor threshold of the user using the magnetic field conversion module.

8. The brain stimulating device including a navigation device for guiding a position of a coil of claim 7, comprising:
an RC controller for receiving location data calculated by the location data calculating unit and controlling a movement direction or a rotation axis of a motor of the robot arm according to the location data.

9. The brain stimulating device including a navigation device for guiding a position of a coil of claim 8, wherein the RC controller is configured to map a zero point of the robot arm to a zero point of the mapped image, and drive the robot arm on 5 axes or more, and
dispose the robot arm at a mapped zero point before moving the robot arm.

10. The brain stimulating device including a navigation device for guiding a position of a coil of claim 7, further comprising:
a cognitive training device for performing cognitive training by setting a preset problem corresponding to a brain region so as to activate the brain region stimulated by the magnetic field generating device, after brain stimulation by the magnetic field generating device in conjunction with the magnetic field generating device, or
an integrated operator connected to the magnetic field generating device and the cognitive training device through communication, and driving the magnetic field generating device and the cognitive training device simultaneously or alternately.

11. The brain stimulating device including a navigation device for guiding a position of a coil of claim 7, further comprising:
a motor threshold control module for disposing the coil in a head position corresponding to a position of the motor cortex in the brain region,
wherein the motor threshold control module drives the RC controller so that the robot arm moves in a circle within a predetermined range around the head position corresponding to a determined position of the motor cortex when a motor threshold is not measured.

12. The brain stimulating device including a navigation device for guiding a position of a coil of claim 1, further comprising:
a magnetic field generating device including: a plurality of coils for stimulating a brain region of a user by generating a magnetic field; a helmet-type magnetic stimulating unit in which the plurality of coils are densely arranged in a helmet-type fixing portion worn by the user on his/her head; a magnetic field conversion module for controlling a magnetic field output of the coil by adjusting a magnetic field-related parameter; and a motor threshold measurement module for measuring a motor threshold of the user using the magnetic field conversion module.

13. The brain stimulating device including a navigation device for guiding a position of a coil of claim 12, wherein the navigation device further comprises:
a multicoil controller for selecting at least one of the plurality of coils according to location data calculated by the location data calculating unit, and transmitting an identification mark of a selected coil to the magnetic field generating device.

14. The brain stimulating device including a navigation device for guiding a position of a coil of claim 13, wherein the multicoil controller controls switching of switches connected to each of the coils to determine whether each of the coils is switched on or off.

15. The brain stimulating device including a navigation device for guiding a position of a coil of claim 13, wherein the coils are connected in the form of an eight-shaped coil, or each of the coils are connected to overlap between the coils.

16. The brain stimulating device including a navigation device for guiding a position of a coil of claim 12, wherein the helmet-type magnetic stimulating unit includes a calibration bar horizontally disposed in a lower end of the fixing portion, and
the navigation device is configured to,
obtain a tilted angle of the calibration bar or a distance to a location information point of the camera image using the 3D camera unit,
calibrate the location information point in consideration of calibration data obtained by calculating the angle of the calibration bar or the distance, and
transmit an identification mark of at least one of the plurality of coils disposed corresponding to the brain region to be stimulated, to the magnetic field generating device, based on a calibrated location information point.

17. The brain stimulating device including a navigation device for guiding a position of a coil of claim 12, wherein the navigation device is configured to,
sense an occurrence of a title of the helmet-type magnetic stimulating unit using at least one of a gyro sensor or an acceleration sensor connected to the helmet-type magnetic stimulating unit, and
calibrate a location information point of the camera image in consideration of calibration data in which a tilted angle of the helmet-type magnetic stimulating unit is calculated, and transmit an identification mark of at least one of the plurality of coils disposed corresponding to the brain region to be stimulated, to the magnetic field generating device, based on a calibrated location information point.

18. The brain stimulating device including a navigation device for guiding a position of a coil of claim 13, wherein a motor is connected to at least one of the coils disposed in the helmet-type magnetic stimulating unit, and
the multicoil controller controls the motor so that a coil connected to the motor overlaps at least one of peripheral coils, and determines at least one of whether the coil connected to the motor is moved, a direction of the movement, and a distance of the movement.

19. A method of controlling a navigation device for guiding a position of a coil, the method comprising:
obtaining a 3D camera image by capturing an image of a user by a 3D camera unit;
obtaining a mapped image by mapping the camera image to a 3D medical image including brain region information or mapping the 3D medical image to the camera image; and
calculating real-time 3D coordinates as location data of a brain region to be stimulated in the mapped image,
wherein the calculating real-time 3D coordinates as location data of a brain region to be stimulated, comprises:
detecting a motor cortex and a location of the brain region to be stimulated in a brain region of the medical image,
calculating real-time 3D coordinates for the motor cortex and the location of the brain region to be stimulated from the mapped image, and
providing the coordinates to the magnetic field generating device so that magnetic stimulation may be performed at a magnetic field strength determined according to a motor threshold measured from the calculated coordinates of the motor cortex.

20. A computer-readable recording medium for recording a program for executing the method of claim 19 on a computer.
